# EUROPÄISCHE PATENTSCHRIFT

(11) **EP 0 882 004 B1**
(45) Veröffentlichungstag und Bekanntmachung des Hinweises auf die Patenterteilung: **31.10.2001**
(21) Anmeldenummer: 97903252.1
(22) Anmeldetag: 10.02.1997
(51) Int. Cl.: C07C 17/16, C07C 22/04, C07C 22/00

(54) **VERBESSERTES VERFAHREN ZUR HERSTELLUNG VON HALOGENMETHYLCYCLOPROPANEN UND BESONDERS REINE HALOGENMETHYLCYCLOPROPANE**
IMPROVED PROCESS FOR THE PRODUCTION OF HALOGEN METHYL CYCLOPROPANES AND HIGHLY PURE HALOGEN METHYL CYCLOPROPANES
PROCEDE AMELIORE DE FABRICATON D'HALOGENOMETHYLCYCLOPROPANES ET HALOGENOMETHYLCYCLOPROPANES PARTICULIEREMENT PURS

(30) Priorität: 23.02.1996 DE 19606761
(43) Veröffentlichungstag der Anmeldung: 09.12.1998
(73) Patentinhaber: BAYER AG, 51368 Leverkusen (DE)
(72) Erfinder: KOMOSCHINSKI, Joachim, D-51061 Köln (DE); GEHRING, Reinhold, D-42327 Wuppertal (DE)
(86) Internationale Anmeldenummer: EP9700607
(87) Internationale Veröffentlichungsnummer: WO9730958

(56) Entgegenhaltungen:
- J. ORG. CHEM. (JOCEAH,00223263);84; VOL.49 (3); PP.431-5, UNIV. CONNECTICUT;DEP. CHEM.; STORRS; 06268; CT; USA (US), XP000673291 HRUBIEC R T ET AL: "Regioselective route to sterically hindered cyclopropylcarbinyl halides" in der Anmeldung erwähnt
- CHEMICAL ABSTRACTS, vol. 102, no. 1, 7.Januar 1985 Columbus, Ohio, US; abstract no. 005729, ZHOU Z ET AL: "Synthesis of cyclopropylcarbinyl bromide" XP002030531 & YIYAO GONGYE (YIGODN);84; (5); PP.42-4, SHANGHAI 1ST MED. COLL.;DEP. MED. CHEM.; SHANGHAI; PEOP. REP. CHINA (CN),
- JOURNAL OF ORGANIC CHEMISTRY, Bd. 44, Nr. 16, 1979, EASTON US, Seiten 2842-2845, XP002030530 W.R. DOLBIER ET AL.: "Preparation of benzocyclic(2.2.1)azoxy compounds"

## Beschreibung

Halogenmethylcyclopropane sind wichtige Zwischenprodukte zur Herstellung von Pharmazeutika (siehe z.B. GB-PS 1 136 214, US-PS 3 433 791 und EP-OS 102 833). Wie bei der Herstellung von Pharmazeutika üblich, werden die Zwischenprodukte dafür in möglichst hoher Reinheit benötigt.

Es ist bekannt, daß man Halogenmethylcyclopropane herstellen kann, indem man die entsprechenden Alkohole mit Phosphortrihalogeniden umsetzt. Gemäß Eur. J. Med. Chem.-Chim. Therap. 15, 571 (1980) wird dabei aber bei einer Reaktionstemperatur von -10°C über 70 % unerwünschtes Halogencyclobutan erhalten. Selbst bei den technisch schwierig zu beherrschenden Temperaturen von -65 und -80°C entstehen immer noch 8,0 bzw. 4,3 % unerwünschtes Halogencyclobutan.

Gemäß einem anderen Verfahren werden Halogenmethylcyclopropane durch Umsetzung der entsprechenden Alkohole mit Methansulfonsäurehalogeniden in Gegenwart von Trialkylaminen hergestellt. Dabei muß jedoch eine komplizierte Dosierung und Temperaturführung durchgeführt werden (siehe EP-OS 565 826), wenn man einigermaßen reine Produkte erhalten will. Das erfordert im technischen Maßstab eine aufwendige Meß- und Regeltechnik und eine außergewöhnlich sorgfältige Reaktionsführung.

Man kann Halogenalkyle auch aus Hydroxyalkylen durch Umsetzung mit Arylphosphorhalogeniden herstellen (siehe EP-OS 251 246), wobei man im allgemeinen Triarylphosphordihalogenide einsetzt und in situ daraus mit elementarem Halogen Arylphosphortetrahalogenide herstellt. Dieses Verfahren ist nicht auf die Umsetzung von Hydroxymethylcyclopropanen zu Halogenmethylcyclopropanen angewendet worden. Außerdem sind Triarylphosphordihalogenide nicht gut zugänglich.

Bei dem bisher günstigsten Verfahren gibt man Hydroxymethylcyclopropan zu einem Gemisch aus Hexachloraceton und Triphenylphosphin und erhält so angeblich reines Chlormethylcyclopropan in Ausbeuten von 80 bis 90 % (siehe J. Org. Chem. 49, 431 (1984)). In der gleichen Literaturstelle wird die Herstellung von Brommethylcyclopropan durch Versetzen einer Mischung von Hydroxymethylcyclopropan, Triphenylphosphin und Dimethylformamid mit elementarem Brom beschrieben. Bei einer Reaktionstemperatur von -10°C ist das Produkt angeblich frei von linearen Alkylbromiden und Bromcyclobutan und in 72 %iger Ausbeute erhältlich. Bereits bei Raumtemperatur wird jedoch lineares Brombuten als Hauptprodukt angegeben. Die Nacharbeitung bei -10°C hat ergeben, daß das erhaltene Brommethylcyclopropan 0,6 Gew.-% lineare Verunreinigungen enthält (siehe vorliegendes Vergleichsbeispiel 1). Gemäß der Literaturstelle wird ein ganz geringer Überschuß an Brom eingesetzt (98,9 mmol Brom auf 97,1 mmol Hydroxymethylcyclopropan).

Nachteilig bei diesem Verfahren sind die immer noch unbefriedigende Reinheit der erhaltenen Halogenmethylcyclopropane, die schwierige Zugänglichkeit und Handhabbarkeit von Hexachloraceton und die Verschiedenartigkeit der Herstellungsverfahren für Chlor- und Brommethylcyclopropane. Die Verunreinigung der erhaltenen Halogenmethylcyclopropane mit linearen Verbindungen, insbesondere 1-Halogen-3-butenen, stellt ein besonderes Problem dar, da sich die physikalischen Eigenschaften der linearen Halogenbutene praktisch nicht von denen der entsprechenden Halogenmethylcyclopropane unterscheiden. Deshalb können die unerwünschten linearen Verbindungen mit üblichen Methoden, z.B. durch Destillation, nicht von den gewünschten Halogenmethylcyclopropanen abgetrennt werden.

Es wurde nun ein verbessertes Verfahren zur Herstellung von Halogenmethylcyclopropanen der Formel 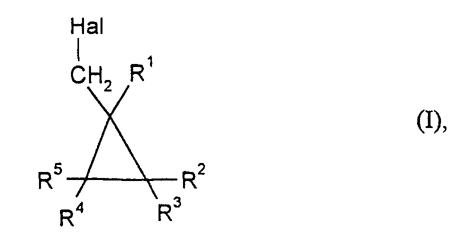 in der
- Hal: für Chlor oder Brom und
- R¹ bis R⁵: unabhängig voneinander jeweils für Wasserstoff, gegebenenfalls substituiertes C₁-C₁₀-Alkyl oder gegebenenfalls substituiertes C₆-C₁₀-Aryl stehen,
gefunden, bei dem man die entsprechenden Hydroxymethylcyclopropane der Formel 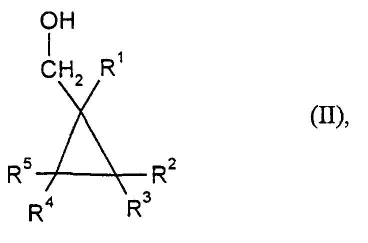 in der
- R¹ bis R⁵: die bei Formel (I) angegebene Bedeutung haben,
mit Chlor oder Brom und einer organischen Phosphorverbindung umsetzt und das dadurch gekennzeichnet ist, daß man Chlor oder Brom in einem Überschuß von wenigstens 5 mol-% (bezogen auf das eingesetzte Hydroxymethylcyclopropan) und als organische Phosphorverbindung eine der Formel (III) einsetzt 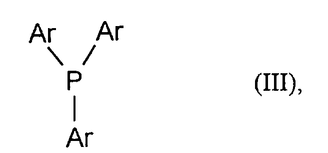 in der
- Ar: unabhängig voneinander jeweils für gegebenenfalls substituiertes C₆-C₁₀-Aryl steht.

Soweit es sich bei den Resten R¹ bis R⁵ um substituiertes C₁-C₁₀-Alkyl handelt kommen als Substituenten beispielsweise Halogen, insbesondere Fluor, Chlor und Brom, und Cyano in Frage. Es können pro Alkylgruppe beispielsweise 1 bis 3 gleiche oder verschiedene Substituenten vorliegen. Die Alkylgruppen können geradkettig oder verzweigt sein.

Soweit es sich bei den Resten R¹ bis R⁵ um substituiertes C₆-C₁₀-Aryl handelt kommen als Substituenten beispielsweise geradkettige und verzweigte C₁-C₆-Alkylgruppen, entsprechende Alkoxygruppen, entsprechende Halogenalkylgruppen, Halogene und Sulfongruppen in Frage. Halogene, auch 'Halogene in Halogenalkylgruppen sind vorzugsweise Fluor, Chlor und/oder Brom. Halogenalkylgruppen können beispielsweise 1 bis 3 gleiche oder verschiedene Halogenatome enthalten. Es können pro Arylgruppe beispielsweise 1 bis 5 gleiche oder verschiedene Substituenten vorliegen.

R¹ bis R⁵ stehen unabhängig voneinander vorzugsweise für Wasserstoff, für C₁-C₄-Alkyl, das gegebenenfalls durch 1 bis 3 Substituenten aus der Gruppe Fluor, Chlor, Brom und Cyano substituiert ist und/oder für Phenyl, das gegebenenfalls durch 1 bis 3 Substituenten aus der Gruppe Methyl, Ethyl, Methoxy, Ethoxy, Chlormethyl, Dichlormethyl, Trichlormethyl, Chlorethyl, Dichlorethyl, Trichlorethyl, Chlor, Brom und Sulfonyl substituiert ist.

Von den Resten R¹ bis R⁵ stehen besonders bevorzugt R¹, R³ und R⁵ für Wasserstoff und R² und R⁴ unabhängig voneinander für Wasserstoff, Methyl, Ethyl, n-Propyl, i-Propyl oder Phenyl. Ganz besonders bevorzugt stehen R¹ bis R⁵ für Wasserstoff.

Die Reste Ar können gegebenenfalls in gleicher Weise substituiert sein wie ober für R¹ bis R⁵ mit der Bedeutung C₆-C₁₀-Aryl angegeben. Besonders bevorzugt sind die Ar-Reste gleich und stehen besonders bevorzugt für Phenyl, Chlorphenyl, Tolyl oder Methoxyphenyl. Ganz besonders bevorzugt stehen die Reste Ar für Phenyl.

Die in das erfindungsgemäße Verfahren einzusetzenden Hydroxymethylpropane der Formel (II) sind bekannte Verbindungen oder können analog zu bekannten Verbindungen hergestellt werden.

Es ist ein wesentliches Merkmal der vorliegenden Erfindung, daß man Chlor oder Brom in einem Überschuß von wenigstens 5 mol-% (bezogen auf eingesetztes Hydroxymethylcyclopropan) einsetzt. Beispielsweise kann dieser Überschuß 5 bis 100 mol-% betragen. Bevorzugt sind Überschüsse von 8 bis 70 mol-%, insbesondere solche von 10 bis 50 mol-%. Durch die Anwendung derartiger Überschüsse erhält man überraschenderweise Halogenmethylcyclopropane der Formel (I), die sich durch einen besonders niedrigen Gehalt an unerwünschten Nebenprodukten auszeichnen. Insbesondere der Gehalt an schwierig abzutrennenden, linearen 1-Halogen-3-butenen liegt bei der erfindungsgemäßen Arbeitsweise im allgemeinen unter 0,4 Gew.-% und kann auf unter 0,1 Gew.-%, gewünschtenfalls auch auf unter 0,01 Gew.-% herabgedrückt werden.

Als organische Phosphorverbindung kann man eine von der Formel (III) umfaßte Einzelverbindung, aber auch ein Gemisch enthaltend mehrere von der Formel (III) umfaßte Einzelverbindungen einsetzen.

Bezogen auf den umzusetzenden Alkohol der Formel (II) kann man organische Phosphorverbindungen der Formel (III) beispielsweise in äquimolaren Mengen oder in Überschüssen bis zur beispielsweise 1,5-fachen äquimolaren Menge einsetzen. Vorzugsweise setzt man die Phosphorverbindung in einem 3 bis 20 mol-%igen, insbesondere 5 bis 15 mol-%igen Überschuß, bezogen auf den Alkohol, ein.

Vorzugsweise führt man das erfindungsgemäße Verfahren in Gegenwart eines Lösungsmittels durch. Geeignet sind insbesondere polare organische Lösungsmittel, beispielsweise Acetonitril, Dimethylformamid, Dimethylacetamid und N-Methylpyrrolidon. Bevorzugt ist Dimethylformamid.

Das erfindungsgemäße Verfahren kann man z.B. bei Temperaturen im Bereich -25 bis +30°C durchführen. Bevorzugt sind -20 bis +25°C, insbesondere -15 bis +5°C.

Die Aufarbeitung des nach Durchführung des erfindungsgemäßen Verfahrens vorliegenden Reaktionsgemisches kann mit an sich bekannten Verfahren erfolgen, z.B. durch Destillation.

Mit dem erfindungsgemäßen Verfahren kann man Halogencyclopropane der Formel (I) in Reinheiten von über 95 %, häufig sogar über 97 % und in Ausbeuten von über 70 %, häufig über 75 % erhalten. Insbesondere ist, wie bereits oben ausgeführt, der Gehalt an störenden und schwierig abtrennbaren linearen 1-Halogen-3-butenen wesentlich geringer als bei bekannten Verfahren. Dies ist besonders überraschend, denn aus dem Stand der Technik konnte nur ein günstiger Einfluß von extrem tiefen Reaktionstemperaturen auf die Zurückdrängung der Bildung von unerwünschten Nebenprodukten entnommen werden, wobei allerdings die mit dem erfindungsgemäßen Verfahren erzielbaren guten Ergebnisse bei weitem nicht erreicht werden.

Die als Nebenprodukt anfallenden Triarylphosphinoxide können auf einfache Weise durch Reduktion in die entsprechenden Triarylphosphine zurückverwandelt und dann erneut als Verbindungen der Formel (III) eingesetzt werden.

Weiterhin betrifft die vorliegende Erfindung Brommethylcyclopropan mit einem Gehalt an offenkettigen Halogenalkenen von weniger als 0,4 Gew.-%, insbesondere weniger als 0,35 Gew.-% und ganz besonders 0,005 bis 0,35 Gew.-%.

### Beispiele

Prozentangaben sind Gewichtsprozente soweit nichts anderes vermerkt ist.

### Beispiel 1 (zum Vergleich)

1250 ml Dimethylformamid wurden vorgelegt, dann 280,8 g Triphenylphosphin und dann 70 g Hydroxymethylcyclopropan zugegeben, 30 Minuten unter Stickstoffatmosphäre bei Raumtemperatur gerührt und anschließend die Lösung auf -10°C abgekühlt. Dann wurden 158,3 g Brom (d.h. 2 mol-% mehr als theoretisch erforderlich) innerhalb von 4 Stunden zudosiert. Die Aufarbeitung des Reaktionsgemisches erfolgte durch Destillation. Brommethylcyclopropan wurde in einer Ausbeute von 77,5 % der Theorie erhalten. Die Reinheit des Produktes lag bei über 97 %, der Anteil an offenkettigen Halogenalkanen bei 0,6 %.

### Beispiel 2

Es wurde verfahren wie in Beispiel 1, jedoch wurde Brom in einem Überschuß von 10 mol-% eingesetzt. Die Ausbeuten und Reinheiten des Produktes waren wie in Beispiel 1 angegeben, der Anteil an offenkettigen Halogenalkenen betrug jedoch nur 0,35 %.

### Beispiel 3

Es wurde verfahren wie in Beispiel 1, jedoch wurde Brom in einem Überschuß von 25 mol-% eingesetzt. Die Ausbeuten und Reinheiten des erhaltenen Produkts entsprachen den in Beispiel 1 angegebenen, der Anteil an offenkettigen Halogenalkanen lag jedoch nur bei 0,071 %.

### Beispiel 4

Es wurde verfahren wie in Beispiel 1, jedoch wurde Brom in einem Überschuß von 50 mol-% eingesetzt. Die erzielten Produktausbeuten und -reinheiten entsprachen den in Beispiel 1 angegebenen, der Anteil an offenkettigen Halogenalkanen lag jedoch nur bei 0,005 %.

### Beispiel 5

Es wurde verfahren wie in Beispiel 2, jedoch wurde statt Brom eine entsprechende Menge Chlor eingesetzt und Chlormethylcyclopropan in entsprechender Ausbeute und Reinheit erhalten.

## Patentansprüche

1. Verfahren zur Herstellung von Halogenmethylcyclopropanen der Formel 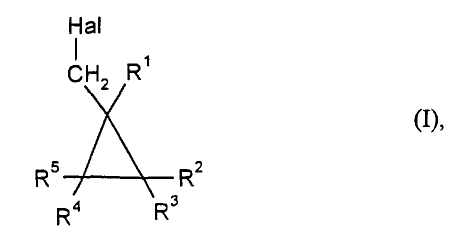 in der
Hal für Chlor oder Brom und
R¹ bis R⁵ unabhängig voneinander jeweils für Wasserstoff, gegebenenfalls substituiertes C₁-C₁₀-Alkyl oder gegebenenfalls substituiertes C₆-C₁₀-Aryl stehen,
bei dem man die entsprechenden Hydroxymethylcyclopropane der Formel 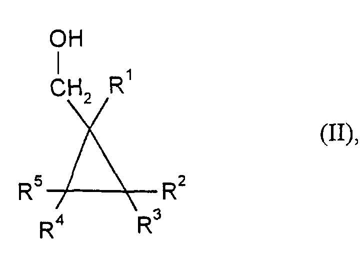 in der
R¹ bis R⁵ die oben bei Formel (I) angegebene Bedeutung haben,
mit Chlor oder Brom und einer organischen Phosphorverbindung umsetzt **dadurch gekennzeichnet, daß** man Chlor oder Brom in einem Überschuß von wenigstens 5 mol-% (bezogen auf das eingesetzte Hydroxymethylcyclopropan) und als organische Phosphorverbindung eine der Formel (III) einsetzt 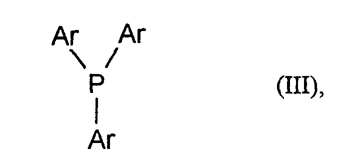 in der
Ar unabhängig voneinander jeweils für gegebenenfalls substituiertes C₆-C₁₀-Aryl steht.

2. Verfahren nach Anspruch 1, **dadurch gekennzeichnet, daß** R¹ bis R⁵ unabhängig voneinander für Wasserstoff, gegebenenfalls durch Halogen und/oder Cyano substituiertes C₁-C₁₀-Alkyl und/oder gegebenenfalls durch geradkettige oder verzweigte C₁-C₆-Alkylgruppen, entsprechende Alkoxygruppen, entsprechende Halogenalkylgruppen, Halogene und/oder Sulfongruppen substituiertes C₆-C₁₀-Aryl und die Reste Ar unabhängig voneinander für gegebenenfalls durch geradkettige oder verzweigte C₁-C₆-Alkylgruppen, entsprechende Alkoxygruppen, entsprechende Halogenalkylgruppen, Halogene und/oder Sulfongruppen substituiertes C₆-C₁₀-Aryl stehen.

3. Verfahren nach Ansprüchen 1 und 2, **dadurch gekennzeichnet, daß** R¹, R³ und R⁵ für Wasserstoff und R² und R⁴ unabhängig voneinander für Wasserstoff, Methyl, Ethyl, n-Propyl, i-Propyl oder Phenyl und die Reste Ar für Phenyl, Chlorphenyl, Tolyl oder Methoxyphenyl stehen.

4. Verfahren nach Ansprüchen 1 bis 3, **dadurch gekennzeichnet, daß** R¹ bis R⁵ für Wasserstoff und die Reste Ar für Phenyl stehen.

5. Verfahren nach Ansprüchen 1 bis 4, **dadurch gekennzeichnet, daß** man Chlor oder Brom in einem Überschuß von 5 bis 100 mol-% einsetzt.

6. Verfahren nach Ansprüchen 1 bis 5, **dadurch gekennzeichnet, daß** man Chlor oder Brom in einem Überschuß von 8 bis 70 mol-% einsetzt.

7. Verfahren nach Ansprüchen 1 bis 6, **dadurch gekennzeichnet, daß** man es in Gegenwart eines polaren organischen Lösungsmittels durchführt.

8. Verfahren nach Ansprüchen 1 bis 7, **dadurch gekennzeichnet, daß** man es in Gegenwart von Acetonitril, Dimethylformamid, Dimethylacetamid oder N-Methylpyrrolidon durchführt.

9. Verfahren nach Ansprüchen 1 bis 8, **dadurch gekennzeichnet, daß** man es im Bereich -25 bis +30°C durchführt.

## Claims

1. Process for the preparation of halogenomethylcyclopropanes of the formula 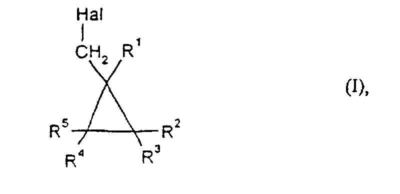 in which
Hal represents chlorine or bromine and
R¹ to R⁵ independently of one another represent in each case hydrogen,
optionally substituted C₁-C₁₀-alkyl or
optionally substituted C₆-C₁₀-aryl
in which the corresponding hydroxymethylcyclopropanes of the formula 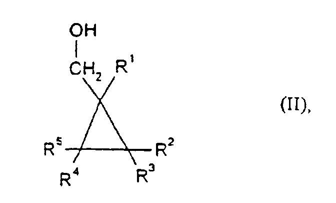 in which
R¹ to R⁵ have the meaning given for formula (I)
are reacted with chlorine or bromine and an organic phosphorus compound and which is **characterized in that** chlorine or bromine is employed in an excess of at least 5 mol% (based on the hydroxymethylcyclopropane employed) and that the organic phosphorus compound employed is one of the formula (III) 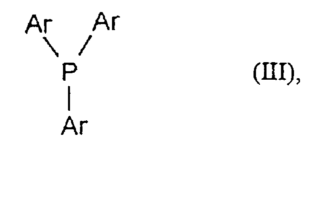 in which
Ar independently of one another represent in each case optionally substituted C₆-C₁₀-aryl.

2. Process according to Claim 1, **characterized in that** R¹ to R⁵ independently of one another represent hydrogen, C₁-C₁₀-alkyl which is optionally substituted by halogen and/or cyano and/or C₆-C₁₀-aryl which is optionally substituted by straight-chain or branched C₁-C₆-alkyl groups, corresponding alkoxy groups, corresponding halogenoalkyl groups, halogens and/or sulpho groups, and the radicals Ar independently of one another represent C₆-C₁₀-aryl which is optionally substituted by straight-chain or branched C₁-C₆-alkyl groups, corresponding alkoxy groups, corresponding halogenoalkyl groups, halogens and/or sulpho groups.

3. Process according to Claims 1 and 2, **characterized in that** R¹, R³ and R⁵ represent hydrogen and R² and R⁴ independently of one another represent hydrogen, methyl, ethyl, n-propyl, i-propyl or phenyl and the radicals Ar represent phenyl, chlorophenyl, tolyl or methoxyphenyl.

4. Process according to Claims 1 to 3, **characterized in that** R¹ to R⁵ represent hydrogen and the radicals Ar represent phenyl.

5. Process according to Claims 1 to 4, **characterized in that** chlorine or bromine is employed in an excess of 5 to 100 mol%.

6. Process according to Claims 1 to 5, **characterized in that** chlorine or bromine is employed in an excess of 8 to 70 mol%.

7. Process according to Claims 1 to 6, **characterized in that** it is carried out in the presence of a polar organic solvent.

8. Process according to Claims 1 to 7, **characterized in that** it is carried out in the presence of acetonitrile, dimethylformamide, dimethylacetamide or N-methylpyrrolidone.

9. Process according to Claims 1 to 8, **characterized in that** it is carried out in the range of -25 to +30°C.

## Revendications

1. Procédé pour la préparation d'halogénométhylcyclopropanes de formule 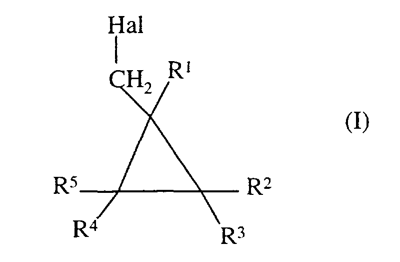 dans laquelle
Hal représente le chlore ou le brome et
R¹ à R⁵ représentent chacun, indépendamment les uns des autres, l'hydrogène, un groupe alkyle en C₁-C₁₀ éventuellement substitué ou un groupe aryle en C₆-C₁₀ éventuellement substitué,
dans lequel on fait réagir les hydroxyméthylcyclopropanes correspondants de formule 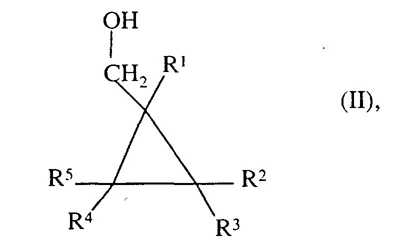 dans laquelle
R¹ à R⁵ ont les significations indiquées en référence à la formule (I),
avec le chlore ou le brome et un composé organique du phosphore, ce procédé se caractérisant en ce que l'on met en oeuvre le chlore ou le brome en excès d'au moins 5 mol % (par rapport à l'hydroxyméthylcyclopropane mis en oeuvre) et on utilise en tant que composé organique du phosphore un composé de formule (III) 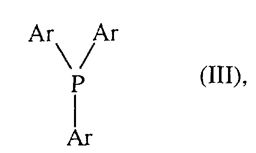 dans laquelle
les symboles Ar représentent chacun, indépendamment les uns des autres, un groupe aryle en C₆-C₁₀ éventuellement substitué.

2. Procédé selon la revendication 1, **caractérisé en ce que** R¹ à R⁵ représentent chacun, indépendamment les uns des autres, l'hydrogène, un groupe alkyle en C₁-C₁₀ éventuellement substitué par des halogènes et/ou des groupes cyano et/ou un groupe aryle en C₆-C₁₀ éventuellement substitué par des groupes alkyle à chaîne droite ou ramifiée en C₁-C₆, les groupes alcoxy correspondants, les groupes halogénoalkyle correspondants, des halogènes et/ou des groupes sulfone, et les symboles Ar représentent chacun, indépendamment les uns des autres, un groupe aryle en C₆-C₁₀ éventuellement substitué par des groupes alkyle à chaîne droite ou ramifiée en C₁-C₆, les groupes alcoxy correspondants, les groupes halogénoalkyle correspondants, des halogènes et/ou des groupes sulfone.

3. Procédé selon les revendications 1 et 2, **caractérisé en ce que** R¹, R³ et R⁵ représentent l'hydrogène et R² et R⁴, indépendamment l'un de l'autre, l'hydrogène, des groupes méthyle, éthyle, n-propyle, isopropyle ou phényle ; et les symboles Ar représentent des groupes phényle, chlorophényle, tolyle ou méthoxyphényle.

4. Procédé selon les revendications 1 à 3, **caractérisé en ce que** R¹ à R⁵ représentent l'hydrogène et les groupes Ar sont des groupes phényle.

5. Procédé selon les revendications 1 à 4, **caractérisé en ce que** l'on met en oeuvre le chlore ou le brome en excès de 5 à 100 mol %.

6. Procédé selon les revendications 1 à 5, **caractérisé en ce que** l'on met en oeuvre le chlore ou le brome en excès de 8 à 70 mol %.

7. Procédé selon les revendications 1 à 6, **caractérisé en ce que** l'on opère en présence d'un solvant organique polaire.

8. Procédé selon les revendications 1 à 7, **caractérisé en ce que** l'on opère en présence de l'acétonitrile, du diméthylformamide, du diméthylacétamide ou de la N-méthylpyrrolidone.

9. Procédé selon les revendications 1 à 8, **caractérisé en ce que** l'on opère dans l'intervalle de température de -25 à +30°C.
